# EUROPEAN PATENT SPECIFICATION

(11) **EP 3 886 831 B1**
(45) Date of publication and mention of the grant of the patent: **06.03.2024**
(21) Application number: 19890140.7
(22) Date of filing: 27.11.2019
(51) Int. Cl.: A61K 31/137, A61K 9/70, A61K 31/445, A61K 9/00, A61K 31/167, A61P 19/00, A61P 23/02, A61K 45/06

(54) **COMPOSITIONS FOR TREATMENT OF SYMPHYSIOLYSIS**
ZUSAMMENSETZUNG ZUR BEHANDLUNG VON SYMPHYSIOLYSE
COMPOSITIONS PERMETTANT LE TRAITEMENT DE LA SYMPHYSIOLYSE

(30) Priority: 28.11.2018 IL 26333618
(43) Date of publication of application: 06.10.2021
(73) Proprietor: Pomp, Gil, 4952708 Petah Tikva (IL)
(72) Inventor: Pomp, Gil, 4952708 Petah Tikva (IL)
(74) Representative: Schulz Junghans Patentanwälte PartGmbB
(86) International application number: PCT/IL2019/051299
(87) International publication number: WO 2020/110118

(56) References cited:
- US-A1- 2009 123 527
- Laboratoire Aguettant: "Package leaflet: Information for the patient", , 1 June 2018 (2018-06-01), pages 1-6, XP055947912, Retrieved from the Internet: URL:https://www.medicines.org.uk/emc/files /pil.12562.pdf [retrieved on 2022-08-01]
- Jar Laboratories: "LIDOPATCH PAIN RELIEF-lidocaine and menthol patch JAR Laboratories", , 1 January 2014 (2014-01-01), pages 1-5, XP055947940, Retrieved from the Internet: URL:https://www.dailymed.nlm.nih.gov/daily med/getFile.cfm?setid=916af96b-84bf-4a1d-a 000-0ccc8ea43ac5&type=pdf [retrieved on 2022-08-01]
- CASTRO ERIC ET AL: "A comparison of transdermal over-the-counter lidocaine 3.6% menthol 1.25%, Rx lidocaine 5% and placebo for back pain and arthritis", PAIN MANAGEMENT, [Online] vol. 7, no. 6, 1 November 2017 (2017-11-01), pages 489-498, XP055947947, GB ISSN: 1758-1869, DOI: 10.2217/pmt-2017-0029 Retrieved from the Internet: URL:http://dx.doi.org/10.2217/pmt-2017-002 9> [retrieved on 2022-08-02]
- VERMANI ERA ET AL: "Pelvic Girdle Pain and Low Back Pain in Pregnancy: A Review", PAIN PRACTICE, vol. 10, no. 1, 1 January 2010 (2010-01-01), pages 60-71, XP055948469, US ISSN: 1530-7085, DOI: 10.1111/j.1533-2500.2009.00327.x
- SARANTEAS THEODOSIOS ET AL: "Ultrasound-Guided Interscalene Brachial Plexus Nerve Block With an Ultralow Volume of Local Anesthetic for Post-Thoracotomy Shoulder Girdle Pain", JOURNAL OF CARDIOTHORACIC AND VASCULAR ANESTHESIA, vol. 32, no. 1, 1 February 2018 (2018-02-01), pages 312-317, XP055948299, AMSTERDAM, NL ISSN: 1053-0770, DOI: 10.1053/j.jvca.2017.04.043
- Broadhurst Norman A. ET AL: "Pain Provocation Tests for the Assessment of Sacroiliac Joint Dysfunction Cite this paper", Journal of spinal disorders, 1 January 1998 (1998-01-01), pages 1-6, XP055949422, Retrieved from the Internet: URL:https://www.academia.edu/28883762/Pain _Provocation_Tests_for_the_Assessment_of_S acroiliac_Joint_Dysfunction?from=cover_pag e [retrieved on 2022-08-05]
- KANAKARIS NK et al.: "Pregnancy-related pelvic girdle pain: an update", BMC Med., vol. 9, no. 15, 15 February 2011 (2011-02-15), pages 1-15, XP021089566, DOI: 10.1186/1741-7015-9-15
- DESMOND FA et al.: "Ultrasound-guided symphysis pubis injection in pregnancy", Anesth. Analg., vol. 111, no. 5, 1 November 2010 (2010-11-01), pages 1329-1330, XP055820518,
- SCICLUNA JK et al.: "Epidural analgesia for acute symphysis pubis dysfunction in the second trimester", Int. J. Obstet. Anesth., vol. 13, no. 1, 31 January 2004 (2004-01-31), pages 50-52, XP055712888,
- SONOHATA M et al.: "Subcutaneous single injection digital block with epinephrine", Anesthesiol. Res. Pract., vol. 487650, 12 July 2001 (2001-07-12), pages 1-4, XP055712892,
- ACHAR S et al.: "Principles of office anesthesia: Part I. Infiltrative anesthesia", Am. Fam. Physician., vol. 66, no. 1, 1 July 2002 (2002-07-01), pages 91-94, XP055340586,
- "Lidocaine and epinephrine injection - FDA prescribing information", , Retrieved from the Internet: URL:http://web.archive.org/web/20160502091 454/https://www.drugs.com/pro/lidocaine-an d-epinephrine-injection.html [retrieved on 2016-05-02]

## Description

### CROSS REFERENCE TO RELATED APPLICATIONS

Benefit is claimed to Israel Patent Application 263336 filed November 28, 2018.

### FIELD

Provided herein are compositions for use in the treatment of symphysiolysis.

### BACKGROUND

Pelvic girdle pain, also known as symphysiolysis, is a common condition stemming from misalignment in the pelvic joints during pregnancy and childbirth. It is associated with pelvic instability, which is relate to increased joint mobility. Symphysiolysis may involve the anterior pelvic joint (symphysis pubis), the posterior pelvic joint (left and right sacroiliac joints) or a combination of them. Symphysiolysis may cause a range of symptoms from pressure or pain in the pelvic region and/or lower abdomen, deferred pain to other areas such as knees or back, or functional loss of leg mobility and functional paralysis. In certain situations, the restriction of movement is so intense as to limit ability to walk and to require use of a walker or wheelchair in order to ambulate.

Clinical bedside diagnosis of symphysiolysis is performed by testing for sensitivity while applying pressure to the symphysis pubis joint, or by using imaging techniques such as x-ray, computerized tomography (CT) and magnetic resonance imaging (MRI).

Accepted treatments include treating with pain killers such as paracetamol and dipyrone, and anti-inflammatory drugs such as non-steroidal anti-inflammatory drugs (NSAIDs) and steroids. However, NSAIDs are not recommended for pregnant women beyond their 30^{th} week of pregnancy, and steroids are considered as drugs of last choice due to maternal side effects. Opioids may be used in post-delivery women, but there is limited safety information relating to effects of long term opioid use during pregnancy. Occasionally, physicians may recommend that patients refrain from certain activities which may move the joint or aggravate the pain, such as climbing stairs. Physicians may also recommend physical therapy or immobilizing the afflicted joint using external orthopedic devices such as supports, braces or girdles. When conservative interventions fail, injections of corticosteroid to the insertion of the sacrospinous ligament on the ischial spine or to the symphysis pubis joint may be performed. In situations that the symphysis pubis joint is dislocated by 4 centimeters or more, surgical intervention may be required to immobilize the joint.

Vermani E et al., reports the intermittent epidural top ups of bupivacaine and fentanyl for 72 h with good results in the relief of PGP. But it also pinpoints the risks of epidural procedure for analgesia in PGP, which include for example tachyphylaxys, motor block, respiratory depression, with consequences for both the mother and the baby (pain practice, vol 10 , n1, pp 60-71). Broadhurst et al.,discloses that the assessment of pain in the vicinity of the sacroiliac joints (where PGP occurs) was performed by PGP provocation pain test before and after the injection of lidocaine intraarticularly in the SIJ (Journal of spinal disorders, vol 11, n4, pp 341-345).

### SUMMARY

The invention is a composition comprising an effective amount of a local anesthetic agent or a salt thereof, for use in subcutaneous administration to a patient i) suffering from pelvic girlde pain or symphysiolysis; and ii) for diagnosis of pelvic girdle pain or symphysiolysis.

The references to methods of treatment in the summary and detailed description are to be interpreted as references to the compounds, pharmaceutical compositions and medicaments of the present invention for use in a method for treatment of the human body by therapy and diagnosis.

Described herein are methods for treatment of symphysiolysis or pelvic girdle pain, comprising administering an effective amount of a local anesthetic agent to a patient in need thereof, to block neural transmission at the inguinal region, optionally at the superficial inguinal ring or canal, or at the anterior superior iliac spine (ASIS) pelvic landmark.

The administration is through the subcutaneous route. Optionally, the subcutaneous administration is administered to a region between the symphysis pubis and the anterior superior iliac spine.

Additionally described herein are compositions comprising an effective amount of a local anesthetic agent for blocking neural transmission at the superficial inguinal ring or canal, for treatment of symphysiolysis. Optionally, the compositions are for administration through the subcutaneous route. Optionally, the compositions are for administrations to a region between the symphysis pubis and the anterior superior iliac spine. The region may be in proximity to the inguinal canal.

Also provided herein are kits comprising packaged pharmaceutical compositions, the compositions comprising an anesthetic agent; and written instructions, the instructions directing to administer to a patient suffering from symphysiolysis the pharmaceutical compositions through the subcutaneous route to block neural transmission at the superficial inguinal ring or canal.

The foregoing and other objects, features, and advantages will become more apparent from the following detailed description, which proceeds with reference to the accompanying figures.

### BRIEF DESCRIPTION OF THE DRAWINGS

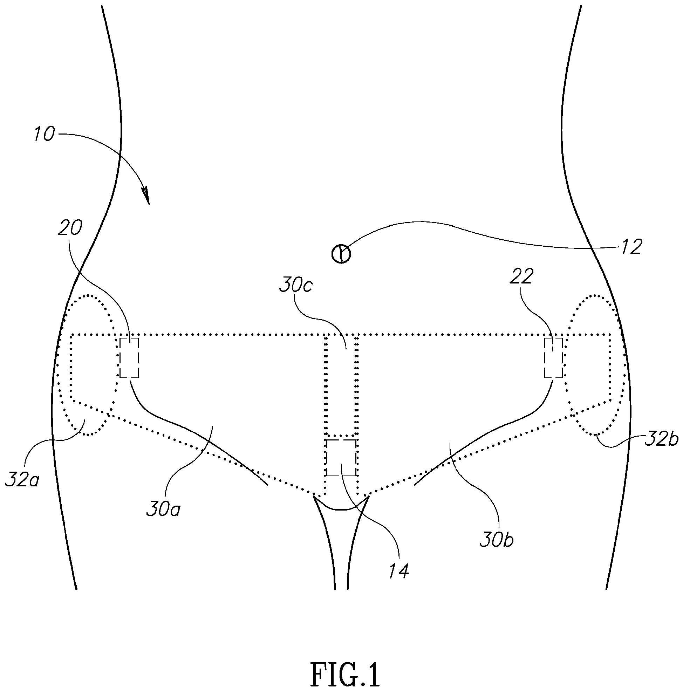

**Figure 1** is a schematic diagram showing the external anatomy of a female abdomen and regions for administration of compositions described herein.

### DETAILED DESCRIPTION

### Terms

Unless otherwise noted, technical terms are used according to conventional usage.

Unless otherwise explained, all technical and scientific terms used herein have the same meaning as commonly understood by one of ordinary skill in the art to which this disclosure **belongs. The singular terms "a," "an," and "the" include plural referents unless context clearly indicates otherwise. Similarly, the word "or" is intended to include "and" unless the context** clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, **suitable methods and materials are described below. The term "comprises" means "includes." The abbreviation,** "*e.g*." **is de**rived from the Latin *exempli gratia* and is used herein to indicate a non**-limiting example. Thus, the abbreviation** "*e.g*." **is synonymous with the term "for example."**

In case of conflict, the present specification, including explanations of terms, will control. In addition, all the materials, methods, and examples are illustrative and not intended to be limiting.

**Anesthetic Agent:** A drug administered to induce temporary loss of sensation.

**Inguinal Canal:** a small passage, about 4-6 cm long, that extends medially and inferiorly through the lower part of the abdominal wall located just above the inguinal ligament, is.

**Local Anesthetic Agent:** A drug administered to a part of a body to induce temporary loss of sensation in a part of the body.

**Subcutaneous administration:** administration of a drug in a bolus to the subcutis, below the dermis.

**Symphysiolysis:** a condition causing pain or discomfort in women, stemming from misalignment in the symphysis pubis joints during pregnancy and childbirth. It may also be known as symphysis pubic dysfunction, symphysis pubis separation, symphysis pubis pain, pubic diastasis, osteitis pubis and pelvic girdle pain - anterior.

### III. Overview of Several Embodiments

Provided herein are compositions and kits comprising an anesthetic agent for use in the treatment of symphysiolysis. The anesthetic agent is a local anesthetic agent. Further provided herein are methods for treatment comprising administering local anesthetic agents.

The anesthetic agent, according to an embodiment, may be a compound or a salt thereof, selected from a group consisting of amino-ester anesthetics and amino-amide anesthetic.

An amino-ester anesthetic may have the basic structural formula: 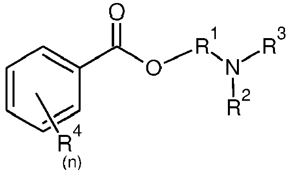
Wherein R¹ is C₁ to C₄ alkyl;
R² and R³ are each independently hydrogen, C₁-C₄ alkyl, or together form a cycloalkyl ring, the ring may be substituted by lower alkyl
R⁴ is amino, halogeno, C₁-C₄ alkyl, C₁-C₆ alkoxy, or C₁-C₆ alkylamine; and
n is between 0 and 5.

The anesthetic may be an amino-ester anesthetic or a pharmaceutically acceptable salt thereof described in table 1.

**Table 1:**

| **Anesthetic name** | **Structure** |
|---|---|
| Benzocaine | 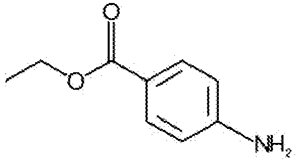 |
| Chloroprocaine | 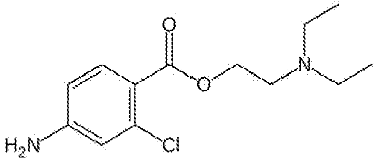 |
| Cyclomethycaine | 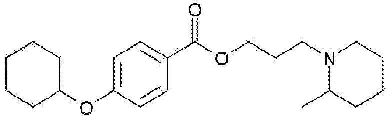 |
| Piperocaine | 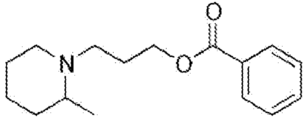 |
| Propoxycaine | 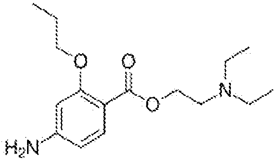 |
| Procaine | 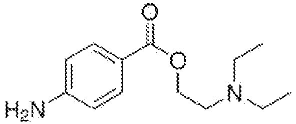 |
| Proxymetacaine | 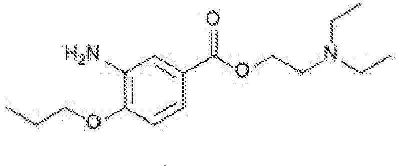 |
| Tetracaine | 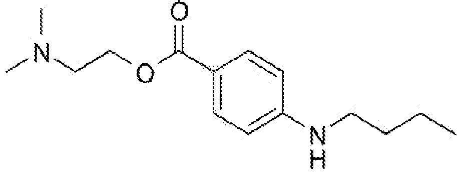 |

The anesthetic agent, according to an embodiment, may be an amino-amide anesthetic or a salt thereof.
An amino-amide anesthetic may have the basic structural formula: 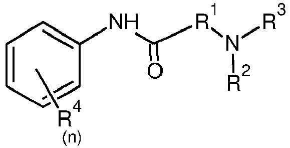
Wherein R¹ is C₁ to C₄ alkyl;
R² and R³ are each independently hydrogen, C₁-C₄ alkyl, or together form a cycloalkyl ring, the ring may be substituted by lower alkyl, or R¹ and R³ together form a cycloakyl ring;
R⁴ is amino, halogeno, C₁-C₄ alkyl, C₁-C₆ alkoxy, or C₁-C₆ alkylamine; and n is between 0 and 5.
The anesthetic may be an amino-amide anesthetic described in table 2 or a pharmaceutically acceptable salt thereof.

**Table 2:**

| Anesthetic Name | Structure |
|---|---|
| Articaine | 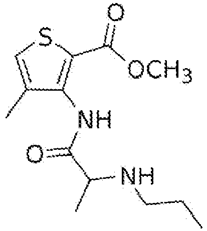 |
| Bupivicaine | 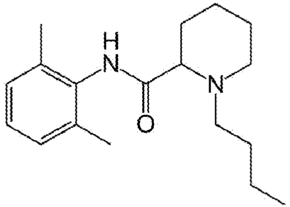 |
| Cinchocaine | 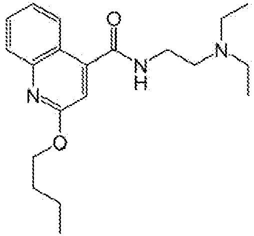 |
| Etidocaine | 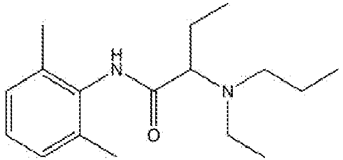 |
| Levobupivacaine | 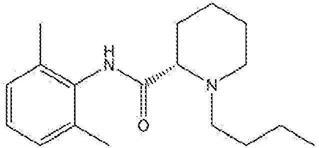 |
| Lidocaine | 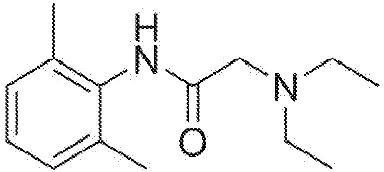 |
| Mepivacaine | 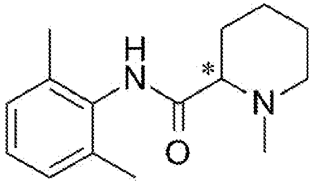 |
| Prilocaine | 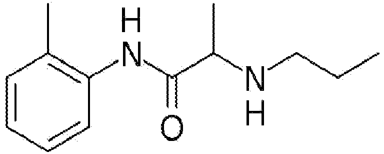 |
| Ropivacaine | 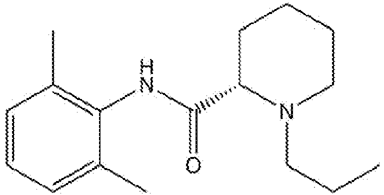 |
| Trimecaine | 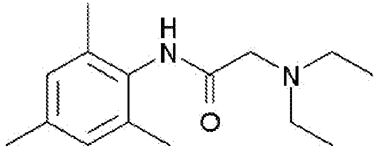 |

According to an embodiment, the anesthetic used may be a short-acting, intermediate-acting or long-acting local anesthetic. Optionally, a patient may be treated with a combination of anesthetics of different durations. Exemplary intermediate-acting anesthetic agents are: lidocaine, mepivacaine and prilocaine. Exemplary long-acting anesthetic agents are bupivacaine and etidocaine.

According to an embodiment, a local anesthetic agent is administered in combination with an adjuvant. The adjuvant may be selected from the group consisting of: a vasoconstrictor, a pH adjusting agent, and a viscosity modifying agent.

According to an embodiment, a local anesthetic agent is administered in combination with a vasoconstrictor. An exemplary vasoconstrictor used in combination with a local anesthetic is epinephrine. Combination of a local anesthetic with a vasoconstrictor may increase the duration of the nerve blocking effect of the local anesthetic.

According to an embodiment, a local anesthetic agent is administered with a pH adjusting moiety. The pH adjusting moiety may be a buffer. The pH adjusting moiety may impact the initiation or duration of activity of the local anesthetic. According to an embodiment, the pH adjusting moiety is sodium bicarbonate.

According to an embodiment, a local anesthetic agent is administered in combination with a viscosity modifying agent. Optionally, the viscosity modifying agent increases the specific gravity of the composition. The composition's modified specific gravity may be above 1.02 upon addition of the viscosity modifying agent. Optionally, the viscosity modifying agent is a sugar. The sugar may be dextrose. The sugar may be added in an amount of between 1% and 50%, preferably between 5% and 10%.

Reference is now made to Fig. 1, which depicts a schematic diagram showing the external anatomy of a female 10 abdomen. Female 10 may be a woman suffering from symphysiolysis. Female 10 may be pregnant, optionally in week 32 or greater of pregnancy. Optionally, female 10 may have given birth within the last month. Female 10 comprises a navel 12, a symphysis pubis 14, a right anterior superior iliac spine 20, and a left anterior superior iliac spine 22. Inguinal region 30 is depicted with a dotted line. Inguinal region 30 comprises right inguinal region 30a, left inguinal region 30b and superior inguinal region 30c. Female 10 comprises a right ASIS region 32a and a left ASIS region 32b, collectively designated as ASIS region.

Inguinal region is where administration is recommended according to some embodiments. Optionally, administration may be through subcutaneous injection in the inguinal region. Optionally, the administration may be performed by injection to left inguinal region 30b or to right inguinal region 30a, or to the superior inguinal region 30c. Optionally, the administration may be performed by injection to both right inguinal region 30a and left inguinal region 30b. Optionally, the administration may be performed by injection to all three inguinal regions 30a, 30b and 30c. Optionally, the physician may penetrate the skin outside inguinal region 30 to administer subcutaneously under inguinal region 30.

ASIS region is where administration is recommended according to some embodiments. Optionally, administration may be through subcutaneous injection in the ASIS region. Optionally, the administration may be performed by injection to left ASIS region 32b or to right ASIS region 32a. Optionally, the administration may be performed by injection to both right ASIS region 32a and left ASIS region 32b. Optionally, the physician may penetrate the skin outside ASIS region 32 to administer subcutaneously under ASIS region 32.

Inguinal region for administration may run from the lateral side of the symphysis pubis to the anterior superior iliac spine, optionally laterally for 5 centimeters from the anterior superior iliac spine. In the superior direction, the inguinal region continues to a lateral line midway between the symphysis pubis and the navel. In the inferior direction, the inguinal region continues 2 cm inferior to the inguinal ligament.

According to an embodiment, the dose of local anesthetics administered is between 10% of the maximal dose and the maximal dose. Maximal dose for exemplary anesthetic agents is described in Table 3:

**Table 3:**

| Anesthetic agent | Maximal dose in milligram per kilogram bodyweight of patient |
|---|---|
| Lidocaine | 4.5 |
| Mepivacaine | 4 |
| Prilocaine | 7 |
| Bupivacaine | 2.5 |
| Etidocaine | 4 |

According to an embodiment, a patient suffering from symphysiolysis may be treated with a test dose, comprising a test amount of a local anesthetic agent. If the outcome of the administration of the test dose shows improvement in patient symptoms, the dosage may be maintained, or may be increased in future administrations. According to an embodiment, the test dose may comprise between 10 and 50 percent of the maximal dose of an anesthetic. The test dose may then be followed by a treatment dose. The treatment dose may comprise between 50 and 100 percent of the maximal dose of an anesthetic. Similarly, a kit may be prepared, the kit comprising a plurality of packaged dosage forms wherein at least one dosage form comprises a test dose and at least one dosage form comprises a treatment dose. The kit may further comprise written instructions, the instructions directing to administer to a patient suffering from symphysiolysis the pharmaceutical compositions through the subcutaneous or transdermal route to block neural transmission at the superficial inguinal ring or canal. Transdermal route is an aspect not covered by the claims.

The kit for use in the treatment of symphysiolysis may comprise between 2 and 30 packaged dosage forms. The packaged dosage forms may each comprise one anesthetic agent. Optionally, the packaged dosage forms may each comprise at least two anesthetic agents. The at least two anesthetic agents comprised in each packaged dosage form may comprise an intermediate-acting anesthetic and a long-acting local anesthetic.

According to an embodiment, the intermediate acting anesthetic may be administered before the administration of the long-acting anesthetic.

According to an embodiment, the anesthetic is administered in a volume of between about 1-20 ml per administration. Optionally, the anesthetic is administered in an amount between 2.5-20 ml per administration.

According to an embodiment, a dosage form comprising a local anesthetic is administered to a patient once daily. According to an embodiment, dosage forms comprising a local anesthetic are administered to a patient twice daily.

According to an embodiment, the dosage form is a pre-filled syringe for subcutaneous administration. Optionally, the syringe is equipped with a needle between 1 and 2 centimeters (cm) in length. Optionally, the needle is between 1.2 and 1.6 cm in length. Optionally, the needle is between 26 Gauge (G according to Birmingham Gauge) and 32G . Optionally, the needle diameter is less than 0.5 millimeters (mm).

According to an embodiment, a dosage form is in the form of a cartridge comprising a local anesthetic for use in combination with a pump for subcutaneous administration. The pump may be configured to be secured outside of the body of a patient and loaded with a cartridge. The pump may be flow-connected to an infusion set, the infusion set comprising a cannula for subcutaneous insertion. The pump may comprise a control and a power source. The control may be engaged by a patient or a healthcare provider to deliver an amount of the local anesthetic from the cartridge, via the cannula, to the patient.

According to an embodiment, the dosage form is configured for self-injection, allowing a patient to inject herself. The dosage form may be provided in a syringe adapted for self-injection, for example by an Autoject device (available from Owen Mumford Inc., \Oxfordshire, United Kingdom) or an Intevia ^{™} handheld autoinjector or a Vystra^{™} disposable pen (both available from BD Medical - Pharmaceutical Systems, Franklin Lakes, New Jersey, United States).

According to an embodiment, the dosage form is a vial. According to a non-claimed aspect of the invention the dosage form is a patch for transdermal administration.

The following examples are provided to illustrate certain particular features and/or embodiments. These examples should not be construed to limit the disclosure to the particular features or embodiments described.

### EXAMPLES

### Example 1:

A 30 year old female patient weighing about 60 kilograms had previously suffered from a car accident with injury to her left leg, resulting in complex regional pain syndrome. Walking was impaired, and crutches were required for mobility. During pregnancy, she was diagnosed with symphysiolysis and she was confined to a wheelchair as she was unable to ambulate using crutches. Treatment including analgesics, NSAIDs, bedrest and external pelvic immobilizations were all attempted but without success.

Subcutaneous lidocaine HCl, 1%, was administered to the inguinal region, in an amount of 5 ml, diluted with another 5 ml of saline solution, to each side of the inguinal region. After administration, the patient was able to stand without pain and no longer required a wheelchair. The treatment lasted for one hour. After one hour, additional Bupivacaine HCl was administered, 0.5% of an amount of 5 ml, diluted in 5 ml of saline solution to each side of the inguinal region. The patient had mobility for an additional nine hours.

Before and after the administration ultrasound to test fetal pulse was performed to confirm that there was a fetal pulse. The patient was directed to perform fetal monitoring on the day after the administration.

### Example 2:

A 31-year-old woman, weighing about 80 kilograms, was in her 33^{rd} week of pregnancy when she was diagnosed with symphysiolysis by the physician, by detecting significant sensitivity at the symphysis pubis joint. She was generally healthy and did not take medications regularly. She had no previous surgeries. She had 3 previous childbirths. Her first childbirth was in week 39, fetal weight 3750 grams. Her second childbirth was in week 39, with a fetal weight of 3850 grams. Her third childbirth was in week 41, with a fetal weight of 4100 grams. She was not diabetic. She was recommended to perform orthopedic support, physical therapy and to medicate with oral paracetamol and dipyrone for pain management. Bedrest was recommended.

At 35 weeks she felt an increase in the severity of symphysiolysis. She used orthopedic support methods which were not sufficient to relieve the symptoms. She had considered inducing labor and using a walker to enhance mobility.

She received 5 milliliters (ml) of lidocaine HCl 1%, diluted in 5% saline, injected subcutaneously to the inguinal region, on both right and left sides.

The patient reported a gradual relief of symphysiolysis symptoms between 10 minutes and 30 minutes from the time of injection. After 30 minutes, there was significant relief. The previously reported sharp pain in the left groin area disappeared. Pain in hip joint which had previously limited walking was significantly reduced. The patient was able climb stairs without significant pain. Stability in walking was increased.

The patient also reported slight dizziness and occasionally heat rate increases after the treatment.

### Example 3:

A 31-year-old woman, weighing about 60 kilograms, in her 20^{th} week complained of groin pains which spread to her right leg. She had difficulty walking and appeared to be dragging her right leg. Upon examination, no sensitivity in symphysis pubis joint was evident. Sensitivity was noticeable at the right sacroiliac joint. Reducing physical activity was recommended by the physician.

After about six weeks, the patient returned with noticeable sensitivity at the left sacroiliac joint, and some sensitivity at the right sacroiliac joint. Painkillers and orthopedic stabilization of the hip were recommended. NSAIDs were prescribed to be used until the 31^{st} week. The patient had symptoms of symphysiolysis in the 29^{th} week which were relieved using NSAID therapy (diclofenac sodium).

During the 31^{st} week, the patient felt an increase in symphysiolysis. She did not require a walker. The patient complained of temporary paralysis during walking, difficulty sleeping and difficulty sitting because of pain in the groin and back areas. Subcutaneous anesthesia to the inguinal region was recommended. A fetal ultrasound was performed, and fetal movement and pulse were both satisfactory.

The patient was injected, subcutaneously in the inguinal region with 1% lidocaine. Three hours after treatment, the patient felt an improvement in pain and in walking but still felt minor freezing of gait. Six hours after treatment, the patient felt less of an influence of the anesthetic, but still felt that walking was better than before the treatment. On the next day, 20 hours after treatment, the patient said that back pains returned, but to a lesser extent than before the treatment. The patient indicated that sleeping was greatly improved.

Two days post lidocaine treatment, the patient felt increased pain, and paresthesia in the legs. That day, the patient received 1 ml of lidocaine 1%, without saline, subcutaneously to the inguinal region, on both right and left sides. After 5 minutes, there was an improvement in walking, but pain still remained. Ten minutes after the lidocaine administration, the patient was able to walk without pain and did not feel back pain. Twenty minutes after the lidocaine administration, the patient received bupivacaine, 0.5%, in an amount of 5 ml to both sides (not diluted with saline), via subcutaneous administration to the inguinal region. Three hours after lidocaine administration, the patient reported a significant improvement in walking, decrease in pain, and increase in walking speed. The patient reported fatigue. Five hours after lidocaine administration, the patient felt a further improvement in pain level.

As shown in the examples, subcutaneous administration of local anesthetic to the inguinal region in pregnant women suffering symphysiolysis decreases pain in walking and other activities.

In addition, methods for diagnosis of a patient suffering from symphysiolysis are also described, according to some embodiments. A patient may show a symptom of symphysiolysis. In order for a physician to determine if the patient is suffering from symphysiolysis, the physician may administer to the patient an amount of a local anesthetic agent to the patient. If the result of the administration is an improvement in a symptom, the patient may be diagnosed with symphysiolysis, and appropriate treatment may be administered. Optionally, the administration is a subcutaneous at the superficial inguinal ring or canal. Optionally, the subcutaneous administration is administered to a region between the symphysis pubis and the anterior superior iliac spine.

Embodiments described herein relate to a composition comprising an effective amount of a local anesthetic agent or a salt thereof, for use in subcutaneous administration to a patient suffering from pelvic girdle pain or symphysiolysis. Optionally, the composition is for use in blocking neural transmission at the superficial inguinal ring or canal, or ASIS region. Optionally the compositions is for use in administration to the inguinal region, or ASIS region. Optionally, the composition is for use in administration to the left inguinal region, the right inguinal region, the superior inguinal region or any combination of the superior, left and right inguinal regions, or ASIS region. Optionally, the patient is a pregnant woman, optionally in the 30^{th} or greater week of pregnancy. Optionally, the patient is post pregnancy. Optionally, the local anesthetic agent or salt thereof comprises an intermediate-acting or long-acting local anesthetic. Optionally, the composition comprises an intermediate-acting and a long-acting local anesthetic or salt thereof. Optionally, the local anesthetic is selected from a group consisting of amino-ester anesthetics and amino-amide anesthetic and salts thereof. Optionally, the local anesthetic is an amino-amide anesthetic or a salt thereof. Optionally, the composition further comprises a vasoconstrictor. Optionally, the vasoconstrictor is epinephrine. Optionally, the composition further comprises a pH adjusting moiety. Optionally, the pH adjusting moiety is sodium bicarbonate. Optionally, the compositions further comprises a viscosity modifying agent. Optionally, the specific gravity is above 1.02. Optionally, the viscosity modifying agent is present in the composition in an amount of between 1% and 50%. Optionally, the local anesthetic agent is lidocaine. Optionally, the local anesthetic agent is bupivicaine. Optionally, the compositions is for administration in the region between the symphysis pubis and the anterior superior iliac spine. Optionally, the composition provides nerve blocking effect for between 6 and 24 hours.

Some embodiments described herein relate to a single-use container comprising a composition for use in the treatment of symphysiolysis, according to any one of the previous claims. Optionally, the container is in the form of a prefilled syringe. Optionally, the container is in the form of a cartridge for use in combination with a pump for subcutaneous administration. Optionally, the container comprises a volume of composition of between 1 and 20 ml. Optionally, the container comprises a volume of composition of between 2.5 and 20 ml. Optionally, the container comprises between 5 and 400 mg of a local anesthetic agent. Optionally, the container comprises between 50 and 200 mg of a local anesthetic agent. Optionally, the container comprises between 10% and 50% of the maximal dose of the anesthetic. Optionally, the container comprises between 50% and 100% of the maximal dose of the anesthetic. Optionally, a kit for use in the treatment of symphysiolysis comprising two or more single-use containers is provided. Optionally, the kit according further comprises written instructions, instructing to administer to a patient suffering from pelvic girdle pain or symphysiolysis. Optionally, the instructions direct a user of the kit to administer a composition to the inguinal region. Optionally, the kit comprises between 2 and 30 single-use containers. Optionally, the kit comprises at least two single-use containers, wherein at least one single-use container comprises a first dose and at least one single-use container in the kit comprises a second dose, and wherein the first dose is different than the second dose. Optionally, the first dose is a test dose and the second dose is a treatment dose.

Some embodiments described herein relate to a composition comprising an effective amount of a local anesthetic agent or a salt thereof, for use in subcutaneous administration to a patient for diagnosis of pelvic girdle pain or symphysiolysis. Optionally, the composition is for use in blocking neural transmission at the superficial inguinal ring or canal or ASIS region. Optionally, the compositions is for use in administration to the inguinal region or ASIS region.

Some embodiments described herein relate to a method for treatment of a patient suffering from pelvic girdle pain or symphysiolysis comprising administering to the patient a composition comprising an effective amount of a local anesthetic agent or a salt thereof, via subcutaneous administration. Optionally, the composition blocks neural transmission at the superficial inguinal ring or canal, or ASIS region. Optionally, the composition is administered to the inguinal region, or ASIS region. Optionally, the composition is administered to the left inguinal region, the right inguinal region, the superior inguinal region or any combination of the superior, left and right inguinal regions, or ASIS region. Optionally, the patient is a pregnant woman. Optionally, the patient is in the 30^{th} or greater week of pregnancy. Optionally, the patient is post pregnancy. Optionally, the local anesthetic agent or salt thereof comprises an intermediate-acting or long-acting local anesthetic. Optionally, the method comprises administering an intermediate-acting and a long-acting local anesthetic or salt thereof. Optionally, the local anesthetic is selected from a group consisting of amino-ester anesthetics and amino-amide anesthetic and salts thereof. Optionally, the local anesthetic is an amino-amide anesthetic or a salt thereof. Optionally, the composition further comprises a vasoconstrictor. Optionally, the vasoconstrictor is epinephrine. Optionally, the composition further comprises a pH adjusting moiety. Optionally, the pH adjusting moiety is sodium bicarbonate. Optionally, the composition further comprises a viscosity modifying Optionally, the viscosity modifying agent is present in the composition in an amount of between 1% and 50%. Optionally, the local anesthetic agent is lidocaine. Optionally, the local anesthetic agent is bupivicaine. Optionally, the composition is administered in the region between the symphysis pubis and the anterior superior iliac spine. Optionally, the composition is provided in a single-use container. Optionally, the composition is in the form of a prefilled syringe. Optionally, the composition is in the form of a cartridge for use in combination with a pump for subcutaneous administration for use in the treatment of symphysiolysis. Optionally, the container comprises a volume of composition of between 1 and 20 ml. Optionally, the container comprises a volume of composition of between 2.5 and 20 ml. Optionally, the patient is administered between 5 and 400 mg of a local anesthetic agent. Optionally, the patient is administered between 50 and 200 mg of a local anesthetic agent. Optionally, the patient is administered between 10% and 50% of the maximal dose of the anesthetic. Optionally, the patient is administered between 50% and 100% of the maximal dose of the anesthetic. Optionally, the administration provides nerve blocking effect for between 6 and 24 hours. Optionally, the composition comprising an effective amount of a local anesthetic agent or a salt thereof, and instructions, the instructions directing the patient suffering from pelvic girdle pain or symphysiolysis to inject the composition via subcutaneous administration. Optionally, the kit for use in the treatment of symphysiolysis comprises two or more single-use containers, each container comprising a composition comprising an effective amount of a local anesthetic agent or a salt thereof. Optionally, the instructions direct a user of the kit to administer a composition to the inguinal region. Optionally, the kit comprises between 2 and 30 single-use containers. Optionally, the kit comprises at least two single-use containers, wherein at least one single-use container comprises a first dose and at least one single-use container in the kit comprises a second dose, and wherein the first dose is different than the second dose. Optionally, the first dose is a test dose and the second dose is a treatment dose.

Some embodiments described herein relate to a method for diagnosis of a patient suffering from pelvic girdle pain or symphysiolysis comprising administering to the patient a composition comprising an effective amount of a local anesthetic agent or a salt thereof, via subcutaneous administration, and determining if the patient suffers from a symptom of pelvic girdle pain or symphysiolysis. Optionally, the composition is administered to block neural transmission at the superficial inguinal ring or canal or ASIS region. Optionally, the composition is administered to the inguinal region or ASIS region.

## Claims

1. A composition comprising an effective amount of a local anesthetic agent or a salt thereof, for use in subcutaneous administration to a patient suffering from pelvic girdle pain or symphysiolysis.

2. The composition according to claim 1 wherein the composition is for use in blocking neural transmission at the superficial inguinal ring or canal, or ASIS region.

3. The composition for use according to claim 1 or 2 wherein the patient is in the 30^{th} or greater week of pregnancy, or post pregnancy.

4. The composition for use according to any one of the previous claims wherein the local anesthetic agent or salt thereof comprises an intermediate-acting or long-acting local anesthetic.

5. The composition for use according to any one of the previous claims wherein the composition further comprises a vasoconstrictor.

6. The composition for use according to any one of the previous claims for administration in the region between the symphysis pubis and the anterior superior iliac spine.

7. The composition for use according to any one of the previous claims wherein the administration provides nerve blocking effect for between 6 and 24 hours.

8. The composition for use according to any one of the previous claims, wherein the composition is provided as a single-use container.

9. The composition for use according to claim 8, wherein the container comprises a volume of composition of between 1 and 20 ml.

10. The composition for use according to claim 8 or 9, wherein the container comprises between 5 and 400 mg of a local anesthetic agent.

11. The composition for use according to any one of claims 8 to 10, wherein the anesthetic agent is selected from lidocaine, mepivacaine, prilocaine, bupivacaine and etidocaine, and wherein the container comprises between 10% and 50% of the maximal dose of the anesthetic given in milligram per kilogram bodyweight of patient according to Table 3:
| Anesthetic agent | Maximal dose in mg per kg |
|---|---|
| Lidocaine | 4.5 |
| Mepivacaine | 4 |
| Prilocaine | 7 |
| Bupivacaine | 2.5 |
| Etidocaine | 4 |

12. The composition for use according to any one of claims 8 to 10, wherein the anesthetic agent is selected from lidocaine, mepivacaine, prilocaine, bupivacaine and etidocaine, and wherein the container comprises between 50% and 100% of the maximal dose of the anesthetic given in milligram per kilogram bodyweight of patient according to Table 3.

13. The composition for use according to any one of the preceding claims 8 to 12, wherein the composition is provided as a kit comprising two or more single-use containers.

14. A composition comprising an effective amount of a local anesthetic agent or a salt thereof, for use in subcutaneous administration to a patient for diagnosis of pelvic girdle pain or symphysiolysis.

15. The composition for use according to any one of the preceding claims, wherein the local anesthetic is administered by intradermal injection into the inguinal region.

## Patentansprüche

1. Eine Zusammensetzung, umfassend eine wirksame Menge eines Lokalanästhetikum-Agens oder eines Salzes davon, zur Verwendung bei subkutaner Verabreichung an einen Patienten, der an Beckengürtelschmerzen oder Symphysiolyse leidet.

2. Die Zusammensetzung gemäß Anspruch 1, wobei die Zusammensetzung zur Verwendung bei der Blockierung der neuronalen Übertragung am oberflächlichen Inguinalring oder-kanal oder der ASIS-Region ist.

3. Die Zusammensetzung zur Verwendung gemäß Anspruch 1 oder 2, wobei die Patientin in der 30. oder höheren Schwangerschaftswoche ist, oder nach Schwangerschaft.

4. Die Zusammensetzung zur Verwendung gemäß einem der vorhergehenden Ansprüche, wobei das Lokalanästhetikum-Agens oder Salz davon ein mittelwirkendes oder langwirkendes Lokalanästhetikum umfasst.

5. Die Zusammensetzung zur Verwendung gemäß einem der vorhergehenden Ansprüche, wobei die Zusammensetzung außerdem einen Vasokonstriktor umfasst.

6. Die Zusammensetzung zur Verwendung gemäß einem der vorhergehenden Ansprüche zur Verabreichung in der Region zwischen der Symphysis pubis und der Spina iliaca anterior superior.

7. Die Zusammensetzung zur Verwendung gemäß einem der vorhergehenden Ansprüche, wobei die Verabreichung eine nervenblockierende Wirkung für zwischen 6 und 24 Stunden bewirkt.

8. Die Zusammensetzung zur Verwendung gemäß einem der vorhergehenden Ansprüche, wobei die Zusammensetzung in einem Einwegbehälter bereitgestellt wird.

9. Die Zusammensetzung zur Verwendung gemäß Anspruch 8, wobei der Behälter ein Volumen von der Zusammensetzung von zwischen 1 und 20 ml umfasst.

10. Die Zusammensetzung zur Verwendung gemäß Anspruch 9 oder 10, wobei der Behälter zwischen 5 und 400 mg von einem Lokalanästhetikum-Agens umfasst.

11. Die Zusammensetzung zur Verwendung gemäß einem der Ansprüche 8 bis 10, wobei das Anästhetikum-Agens ausgewählt ist aus Lidocain, Mepivacain, Prilocain, Bupivacain und Etidocain, und wobei der Behälter zwischen 10 % und 50 % der maximalen Dosis des Anästhetikums, angegeben in Milligramm pro Kilogramm Körpergewicht der Patientin, gemäß Tabelle 3 umfasst:
| Anästhetikum-Agens | maximalen Dosis in mg pro kg |
|---|---|
| Lidocain | 4,5 |
| Mepivacain | 4 |
| Prilocain | 7 |
| Bupivacain | 2,5 |
| Etidocain | 4 |

12. Die Zusammensetzung zur Verwendung gemäß einem der Ansprüche 8 bis 10, wobei das Anästhetikum-Agens ausgewählt ist aus Lidocain, Mepivacain, Prilocain, Bupivacain und Etidocain, und wobei der Behälter zwischen 50 % und 100 % der maximalen Dosis des Anästhetikums, angegeben in Milligramm pro Kilogramm Körpergewicht des Patienten gemäß Tabelle 3, umfasst.

13. Die Zusammensetzung zur Verwendung gemäß einem der vorhergehenden Ansprüche 8 bis 12, wobei die Zusammensetzung als ein Kit bereitgestellt wird, das zwei oder mehr Einwegbehälter umfasst.

14. Eine Zusammensetzung, umfassend eine wirksame Menge eines Lokalanästhetikum-Agens oder eines Salzes davon, zur Verwendung bei subkutaner Verabreichung an einen Patienten zur Diagnose von Beckengürtelschmerzen oder Symphysiolyse.

15. Die Zusammensetzung zur Verwendung gemäß einem der vorhergehenden Ansprüche, wobei das Lokalanästhetikum durch intradermale Injektion in die Inguinalregion verabreicht wird.

## Revendications

1. Composition comprenant une quantité efficace d'un agent anesthésique local ou d'un sel de celui-ci, destinée à être utilisée dans l'administration par voie sous-cutanée à un patient souffrant de douleurs à la ceinture pelvienne ou symphysiolyse.

2. Composition selon la revendication 1, dans laquelle la composition est destinée à être utilisée dans le blocage de transmission neurale au niveau de l'anneau ou du canal inguinal superficiel, ou région ASIS.

3. Composition destinée à être utilisée selon la revendication 1 ou 2, dans laquelle le patient est à la 30^{e} semaine de grossesse ou plus, ou post-grossesse.

4. Composition destinée à être utilisée selon l'une quelconque des revendications précédentes, dans laquelle l'agent anesthésique local ou sel de celui-ci comprend un anesthésique local à action intermédiaire ou longue action.

5. Composition destinée à être utilisée selon l'une quelconque des revendications précédentes, dans laquelle la composition comprend en outre un vasoconstricteur.

6. Composition destinée à être utilisée selon l'une quelconque des revendications précédentes destinée à l'administration dans la région entre la symphyse pubienne et l'épine iliaque antéro-supérieure.

7. Composition destinée à être utilisée selon l'une quelconque des revendications précédentes, dans laquelle l'administration fournit un effet de blocage nerveux pendant une durée comprise entre 6 et 24 heures.

8. Composition destinée à être utilisée selon l'une quelconque des revendications précédentes, dans laquelle la composition est fournie en tant que récipient à usage unique.

9. Composition destinée à être utilisée selon la revendication 8, dans laquelle le récipient comprend un volume de composition compris entre 1 et 20 ml.

10. Composition destinée à être utilisée selon la revendication 8 ou 9, dans laquelle le récipient comprend entre 5 et 400 mg d'un agent anesthésique local.

11. Composition destinée à être utilisée selon l'une quelconque des revendications 8 à 10, dans laquelle l'agent anesthésique est sélectionné parmi la lidocaïne, mépivacaïne, prilocaïne, bupivacaïne et étidocaïne, et dans laquelle le récipient comprend entre 10 % et 50 % de la dose maximale de l'anesthésique donnée en milligramme par kilogramme de poids corporel du patient selon le Tableau 3 :
| Agent anesthésique | Dose maximale en mg par kg |
|---|---|
| Lidocaïne | 4,5 |
| Mépivacaïne | 4 |
| Prilocaïne | 7 |
| Bupivacaïne | 2,5 |
| Étidocaïne | 4 |

12. Composition destinée à être utilisée selon l'une quelconque des revendications 8 à 10, dans laquelle l'agent anesthésique est sélectionné parmi la lidocaïne, mépivacaïne, prilocaïne, bupivacaïne et étidocaïne, et dans laquelle le récipient comprend entre 50 % et 100 % de la dose maximale de l'anesthésique donnée en milligramme par kilogramme de poids corporel du patient selon le Tableau 3.

13. Composition destinée à être utilisée selon l'une quelconque des revendications précédentes 8 à 12, dans laquelle la composition est fournie en tant que kit comprenant deux récipients à usage unique ou plus.

14. Composition comprenant une quantité efficace d'un agent anesthésique local ou d'un sel de celui-ci, destinée à être utilisée dans l'administration par voie sous-cutanée à un patient pour le diagnostic de douleurs à la ceinture pelvienne ou symphysiolyse.

15. Composition destinée à être utilisée selon l'une quelconque des revendications précédentes, dans laquelle l'anesthésique local est administré par injection intradermique dans la région inguinale.
